Europäisches Patentamt

European Patent Office

Office européen des brevets

(11) Publication number: **0 246 638**
**A2**

## (12) EUROPEAN PATENT APPLICATION

(21) Application number: 87107354.0

(22) Date of filing: 20.05.87

(51) Int. Cl.⁴: **A61L 33/00** , A61L 27/00 , A61F 2/06

(30) Priority: 23.05.86 US 866869

(43) Date of publication of application:
25.11.87 Bulletin 87/48

(84) Designated Contracting States:
DE FR GB IT NL

(71) Applicant: Cordis Corporation
10555 West Flagler Street
Miami Florida 33102-5700(US)

(72) Inventor: Halbert, Seymour P.
12450 Rock Garden Lane
Miami Florida 33156(US)

(74) Representative: Kuhnen, Wacker & Partner
Schneggstrasse 3-5 Postfach 1729
D-8050 Freising(DE)

(54) Biologically modified synthetic grafts.

(57) Synthetic porous grafts are provided that have been modified with a conjugate in order to enhace their biocompatibility. The thus modified synthetic grafts have the conjugate formed in place within their pores from collagen materials coupled to anticoagulant materials. Cell attachment protein material may also be included on the conjugate. A method for forming the conjugate in place is also described, as are conjugates incorporating type III collagen fibrils.

FIG. 1

## BIOLOGICALLY MODIFIED SYNTHETIC GRAFTS

### Background and Description of the Invention

The present invention generally relates to synthetic grafts that have a generally porous graft body within at least the pores of which are located a conjugate for enhancing the suitability of the graft for implantation within a living body. More particularly, the conjugate includes collagen components and heparinous material components as well as a suitable crosslinking component. The conjugate achieves biological modification of the synthetic graft in order to enhance its biocompatibility when compared with such a synthetic graft that is not thus subjected to biological modification. Cell attachment protein material can additionally be included on the conjugate in order to further enhance endothelial cell attachment and growth. Included also is a method of making such a biologically modified graft as well as a process for improving biocompatibility and conjugates suitable therefor.

Synthetic or fabricated grafts are known to be made by various methods that are intended to provide structures which provide a degree of porosity that is desired for implantable grafts, especially those that are intended to supply an environment that is particularly suitable for promoting tissue ingrowth under in vivo conditions. Grafts of these types are often intended for use at implantation locations which contact blood flow, such as vascular grafts and the like. Synthetic grafts in this regard include those that are made by methods which include winding extruded material onto a mandrel. This approach is exemplified by United States Letters Patent No. 4,475,972, the disclosure of which is incorporated by reference hereinto. That patent describes non-woven grafts that are made by extruding a polyurethane solution under pressure through an opening and then drawing the extruded material while winding same on the mandrel. Other synthetic grafts include, for example, woven materials, knitted materials, and materials that are rendered porous by salt elution techniques. In its broadest sense, a synthetic graft is one that is made and/or otherwise treated, as distinguished from a graft that is surgically transplanted or relocated.

Whenever such a synthetic graft is implanted, concerns arise that can be generally characterized as those involving biocompatibility. Especially important in this regard is providing a graft-body interface that is more acceptable to the body, in order to significantly improve the clinical acceptability of the grafts. Also important is the encouragement of endothelialization development, particularly at and near those locations of the graft that contact flowing blood. Especially desirable is the development of a complete layer, such as a monolayer, of endothelial cells, this being especially significant to maintaining graft integrity.

When the synthetic graft is one which includes relatively narrow passageways, such as those having inner diameters on the order of about 4 mm or smaller, particular problems develop regarding their ability to function satisfactorily when implanted. Such small passageway synthetic grafts tend to exhibit reduced patency and provide especially low levels of resistance to thrombus formation and blockage. These properties are particularly important when attempting to use synthetic grafts for surgical techniques such as coronary bypass procedures. Luminal thrombus formation with occlusion is a particularly difficult problem that is encountered in the use of such narrow passageway grafts. Patency without embolization is desired.

While the porosity that is provided by these types of synthetic graft structures is extremely desirable and advantageous, a porous synthetic graft is, prior to implantation, typically also porous to the extent that blood or other fluids can pass through the porous wall or porous substrate of the synthetic graft. Typically, before such a porous fabricated graft device is implanted, the surgical team must subject it to preclotting with blood or the like in order to prevent fluid loss upon initial pressurization of the synthetic graft. Such a preclotting step is considered to be a time-consuming annoyance and can provide a potential opportunity for the development of thrombosis and/or infection. Also, the preclotting procedure can vary the environment provided by the synthetic graft depending upon the manner in which the techniques are employed by the individuals carrying out the preclotting procedure, which can affect the reproducibility of synthetic graft implantation techniques.

The problem of attempting to eliminate the need for preclotting of synthetic grafts is rendered more difficult because, for many such grafts, it is important to provide certain porosity properties for both internal and external surfaces thereof. In the example of the tubular synthetic graft, its external cylindrical surface can be rendered porous in order to provide a porous depth that affords a means for fixation to host tissues by soft tissue ingrowth into the porous depth of the surface, while the internal generally cylindrical porous surface affords a porous interface having smaller pore sizes which provide tissue-implant interfaces that are blood compatible arising from colonization and tissue formation on the blood-contacting internal surface of such synthetic grafts. In these instances, tissue ingrowth is desired on the external surface, while endothelial-like cell ingrowth provided by nucleated blood cells or the like is desired on the internal surface. For these reasons, it may be most desirable to provide a synthetic graft that is porous on both of its surfaces, but that still prevents fluid passage from one surface to the other without requiring preclotting or the like.

These concerns which are associated with porous synthetic grafts in general are addressed by the present invention which, in summary, includes a porous synthetic graft having a conjugate that was formed within pores of the graft, which conjugate has properties that significantly modify the biocompatibility aspects of the graft in order to render same more suitable for implantation without rejection and in order to substantially preclude the need for preclotting procedures. Conjugates particularly suitable in this regard are those which include collagen and heparinous material linked together by a suitable crosslinking component such as a dialdehyde. Preferably, the synthetic graft is prepared by a method which includes reconstituting the collagen material and forming the conjugate after the collagen material has been applied into the pores of the graft. In other aspects of the invention, a conjugate is provided that includes type III collagen, and in vivo compatibility between the blood and an implanted porous synthetic graft is improved.

It is accordingly a general object of the present invention to provide an improved biocompatible graft, method of making same, conjugate having biocompatibility enhancement properties, and process including use of same.

Another object of this invention is to provide an improved biocompatible fabricated graft that does not require preclotting, as well as methods for making and using same and conjugates suitable in this regard.

Another object of the present invention is to provide an improved porous synthetic graft, methods of making and using same and conjugates incorporated therewithin which significantly reduce the chance of luminal thrombus formation and occlusion after implantation.

Another object of the present invention is to provide an improved porous synthetic graft, methods and conjugate which, after implantation, encourages new endothelial cell growth for retaining patency and for preventing thrombus formation.

Another object of this invention is to provide an improved porous graft, methods and conjugate for enhancing the ability of endothelial cells to attach and grow on the luminal surface of the graft.

Another object of the present invention is to provide a synthetic porous graft, method of making same, and conjugate for incorporation thereinto which is especially receptive to endothelial growth by itself or in conjunction with grafts seeded with pure endothelial cells grown within a relatively short period of time from a small portion of subcutaneous fat or small vein of the patient.

Another object of this invention is to provide a method for forming a biological enhancement conjugate in place and generally within the pores of a synthetic graft.

Another object of this invention is to provide an improved graft that retains patency even when it takes the form of a graft having an extremely small diameter or other flow path.

Another object of the present invention is to provide an improved synthetic graft having a porous surface for promoting ingrowth under in vivo conditions and which has a biological modifying conjugate that prevents undesired flow through the pores.

Another object of the present invention is to provide an improved porous synthetic graft, method of making same and conjugate used therewith, which graft does not require preclotting prior to implantation.

These and other objects, features and advantages of this invention will be clearly understood through a consideration of the following detailed description.

Brief Description of the Drawings

In the course of this description, reference will be made to the attached drawings, wherein:

Figure 1 is a perspective view, twice partially cut away, of a preferred synthetic porous graft according to the present invention;

Figure 2 is a transverse cross-sectional view of a segment of the graft illustrated in Figure 1;

Figure 3 is a full transverse cross-sectional view along the line 3-3 of Figure 1;

Figure 4 is a transverse cross-sectional view of another embodiment of a synthetic porous graft according to the present invention;

Figure 5 is a generally schematic illustration of an apparatus and method suitable for making the body of the synthetic graft illustrated in Figures 1 through 3;

Figure 6 is a cross-sectional view taken along the line 6-6 of Figure 5; and

Figure 7 is a cross-sectional view taken along the line 7-7 of Figure 6.

## Description of the Particular Embodiments

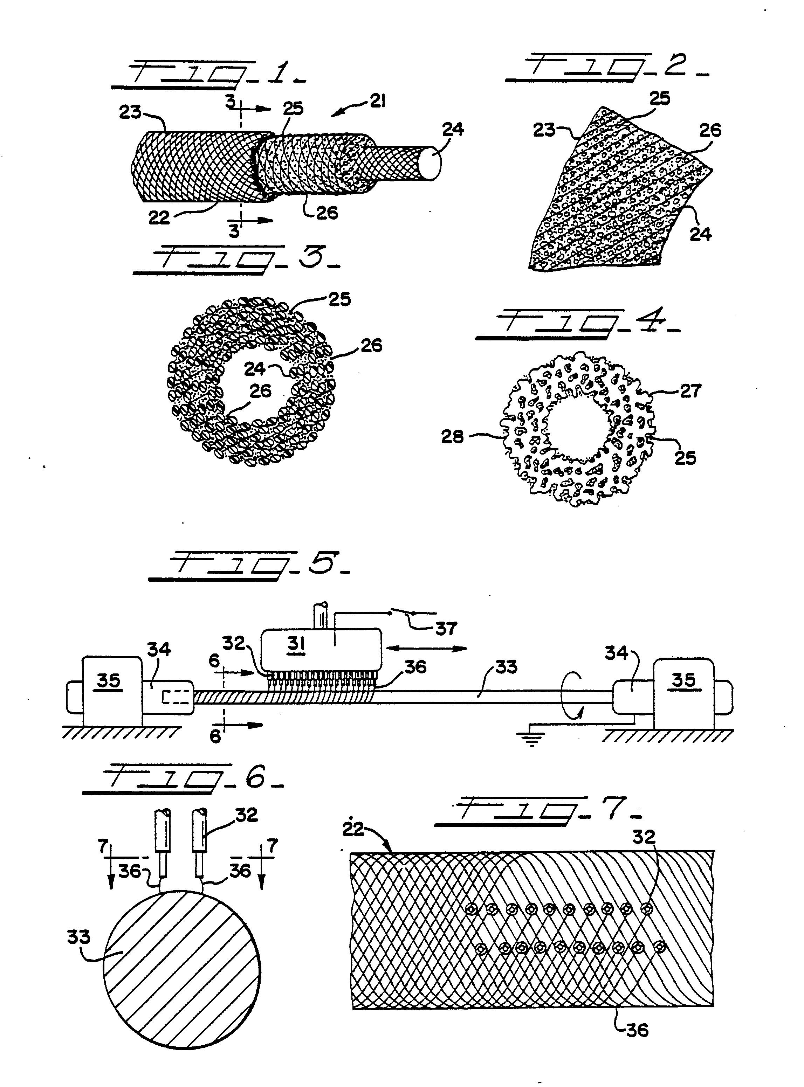

In the embodiment illustrated in Figures 1, 2 and 3, a synthetic graft, generally designated as 21, is shown which includes a graft body 22 having an outer surface 23 and an inner surface or lumen 24. Between the outer surface 23 and the inner surface 24, a conjugate 25 is within pores 26 of the graft body 22. Conjugate 25 forms a generally sheath-like structure within the pores 26 of the body 22, which sheath-like structure includes a network of conjugate material.

Figure 4 illustrates another form of synthetic graft within which conjugate 25 has been formed and is positioned. Graft body 27 includes pores 28 that are formed by eluting or leaching salt particles or the like from a synthetic material. Pores 28 are formed by providing a mixture of polymeric material and elutable particles, such as by extruding them as a sheath or as a layer over a substrate material. The thus formed or shaped polymeric material having elutable particles such as salt granules is subjected to eluting conditions, typically within a suitable solvent such as water, by the operation of which the salt particles dissolve away from the polymeric material in order to form the pores 28 within the polymeric material. Satisfactory elutable particles include salts such as sodium chloride crystals, sodium carbonate, sodium fluoride, magnesium sulfate and other water-soluble materials that are readily leached by the action of water or the like as an elution medium. Other particles that are soluble in organic solvents and the like can be substituted as desired.

Other types of synthetic graft bodies can be utilized provided they exhibit sufficient porosity in order to accept and support the conjugate material therewithin. Typically, such bodies will be made of flexible non-metallic material, including any of various polymeric materials or other organic substances, provided they are substantially inert and/or biocompatible. Suitable polymeric materials include polyurethanes, polycarbonates and various copolymers. When wound, non-woven graft bodies are provided such as those of Figures 1 through 3, the polymeric material should have acceptable fiber-forming properties. Such polymers typically also should possess properties suitable for forming a generally flexible graft, such as reasonable pliability and elasticity as well as biocompatibility.

The apparatus illustrated in Figures 5, 6 and 7 is suitable for providing a non-woven, wound body 22 from which the synthetic graft 21 may be made. Such apparatus includes a distributor 31 for achieving the formation of polymeric fibers from fine orifices 32, typically in conjunction with formation of those fibers from a fiber-forming polymeric solution by generally known extrusion techniques. Before such fibers are fully set, they are wound onto a mandrel 33, which is typically rotated within suitable jaws 34. In the arrangement illustrated, the distributor 31 moves back and forth within a plane generally between the jaws 34, while the mandrel 33 is rotated by a suitable means such as the illustrated motors 35. Alternatively, the distributor 31 can take the form of spinnerette that rotates around the mandrel 33. Whatever mechanism or technique is utilized, such will result in combined rotational and translational relative movement between the mandrel 33 and polymeric fibers 36 flowing from the orifices 32. The illustrated apparatus optionally can include electrostatic charge generation components in order to develop an electrostatic charge between the distributor 31 and the mandrel 33. Mandrel 33 may be grounded or negatively charged, while the distributor 31 is positively charged when a switch 37 or the like is closed. Alternatively, the distributor can be grounded, and the mandrel can be positively charged.

When an apparatus of the type illustrated in Figures 5, 6 and 7 is utilized, the diameter of the inside surface or lumen 24 is substantially the same as the outside diameter of the mandrel 33. Individual fibers 36 are bound to each other, generally at most if not substantially all of the locations where the fibers intersect or otherwise contact each other. This fiber-to-fiber bonding results when the partially set fibers engage one another during winding, which is typically facilitated by drawing the extruded fibers over fibers laying thereunder, such being suitably accomplished by selecting an appropriate speed of relative movement between the mandrel 33 and the distributor 31 which will draw the fibers at a speed that is faster than the rate by which they are extruded from the distributor 31.

With more particular reference to the conjugate material that is present within the pores of the graft body, such be characterized as a modifier of the biological properties of the synthetic graft in order to render same more biocompatible and less likely to develop signs of rejection by the living host within which the synthetic grafts according to this invention are implanted. The conjugate material is instrumental in preventing thrombus formation and encourages endothelial cell growth. Patency retention is enhanced, and the incidence of luminal thrombus formation with occlusion is significantly reduced.

In its broad aspects, the conjugate includes collagen crosslinked or chemically attached to other collagen molecules and to an anticoagulant, particularly a heparinous material. The conjugate formation is best carried out by aldehyde linkages to amino groups. Such linkages include those between an amino group of a collagen molecule and an aldehyde group, as well as between an amino group of heparinous material and an aldehyde group. By utilizing aldehydes having a plurality of aldehyde groups, collagen molecules are thus joined to other collagen molecules and to molecules of the heparinous material. Other similar linkages can be provided, for example, when it is desired to further include a cell attachment protein within the conjugate.

Regarding the collagen material, native collagen exists as fibers in at least five major chemical variants or types. For example, type I collagen is generally associated with mechanical strength, while type III collagen is generally associated with elasticity and flexibility. Native collagen fibers possess a distinct banded structure generally characteristic of a triple helix configuration of the peptide chains and its amino acid composition. Native collagen is relatively insoluble, although it can be solubilized under certain conditions which are generally known, for example at low pH values and/or upon exposure to enzymes such as pepsin. When subsequently thrown out of solution, the collagen automatically reassociates itself back into its native fibrillar form. The present invention includes the recognition of these properties of collagen. It includes forcing solubilized collagen materials into the pores of a synthetic graft, after which they are reformed or reassociated to their native collagen fiber form, such reformation or reassociation taking place within the pores of the synthetic graft and generally around strands or other structural components of the synthetic graft body.

After the collagen solution is positioned within the pores of the graft body, they are treated so that the collagen fibrils reassociate and generally crosslink to each other in order to yield fibers of enhanced stability and structural durability. Compounds having multiple aldehyde groups are particularly well suited for this purpose. The result is a structure by which reassociated native collagen fibrils are stabilized within the pores of the synthetic graft. Because of this reassociation in place and aldehyde crosslinking treatment of the native collagen fibrils, a highly insoluble collagen network is formed that is virtually inert immunologically, thereby substantially reducing or eliminating immunological rejection due to the bovine origins of the collagen.

Continued preparation of the conjugate component of the treated synthetic porous graft includes coupling anticoagulant material onto the formed-in-place matted collagen fibril network. Heparinous materials are particularly desirable anticoagulant materials which inhibit thrombogenesis in order to significantly improve patency prospects after implantation. It is known that heparinous materials in general may be coupled to collagen linking agents including those having aldehyde groups. Often, such heparinous materials used in this regard are mixtures of molecules that exhibit varying specific anticoagulant activities. Such heparinous material mixtures or preparations typically include only about 10% to 20% of the most active heparin fraction in terms of prevention of clot formation, while at least half and usually substantially more than half of the heparinous material fractions in these preparations are fractions that are substantially inactive in terms of clot formation prevention. It is accordingly preferred that the heparinous material of the present invention should include substantially only the more highly active fractions of typical heparinous material preparations. In this regard, primarily the most highly active heparinous material fractions can be isolated by suitable separation techniques such as affinity chromatography on anti-thrombin III sepharose columns.

When desired, the conjugate material of the treated synthetic porous graft may further include cell attachment protein. When the graft is of a generally tubular structure, it is preferred that the cell attachment protein be coupled to the luminal surface of the formed-in-place conjugate. Such cell attachment protein significantly improves new growth of endothelium, especially on the intimal surface of the synthetic graft, after implantation. Such cell attachment protein is for encouraging growth of endothelial cells onto and/or into the graft from the healthy intima at both ends of the graft. This process can be facilitated by seeding the synthetic graft prior to implantation with endothelial cells that preferably were earlier harvested from the patient. Even when such seeding is practiced, minimal portions of subcutaneous fat or a small vein of the patient need to sacrificed for such harvesting and seeding, and it is usually desirable to eliminate or minimize such seeding. An especially suitable cell attachment protein in this regard is fibronectin, highly purified fibronectin being especially suitable. Because of the high affinity of fibronectin for heparinous materials, fibronectin exhibits a spontaneous tendency to link, even without a coupling agent, with the heparinous material and collagen of the conjugate.

Especially suitable coupling or crosslinking agents are those which will readily chemically link with amino groups of the other components that may be present within the conjugate, including the collagen material, the heparinous material, and the cell attachment protein material. Compounds having multiple aldehyde groups are preferred in this regard. Dialdehydes are especially preferred. Exemplary coupling or crosslinking agents have the formula OHC-R-CHO, wherein R, when present, is a carbon chain of at least one carbon atom. The preferred coupling or crosslinking agent is glutaraldehyde because of its commercial availability and the recognition of its ability to be used in conjunction with medical devices.

With more particular reference to the method aspects of this invention, a length of porous synthetic graft is provided and is arranged or oriented for reception of collagen solution into the pores thereof. The collagen solution is formed by dissolving collagen, typically purified bovine collagen, in a suitable solvent. Several different solvent systems are known to be useful in this regard, for example an aqueous buffer that is at an acidic pH. Exposure to pepsin or the like can also be carried out in conjunction with the collagen solution formation. Preferably, the collagen solution is one including type III collagen and may, for example, include a blend of type III collagen with another collagen material such as type I collagen. Other collagen materials are less readily available, such being the type II, type IV and type V collagen fibrils. Thus formed solution is typically in conjunction with imparting pressurized conditions thereto and in order to force the collagen solution thereinto, passed into the pores of the synthetic graft.

Thereafter the thus treated graft is contacted, such as by immersion into a bath, with a reassociating solution that is best suited for effecting collagen reassociation from the particular solvent system that had been used. A reassociating solution that is suitable for use in conjunction with the exemplififed solvent system is a concentrated salt solution. Sodium chloride is a salt that is typically used in this regard.

The porous synthetic graft having the reassociated collagen fibrils therewithin is then subjected to treatment with the crosslinking agent. Typically, the graft is immersed within a bath including a dialdehyde. The bath typically will be slightly alkaline and need contain only low concentrations of the dialdehyde, for example between about 0.5% and about 1% by weight of dialdehyde, based on the total weight of the crosslinking bath. This treatment is continued until such time as the dialdehyde can diffuse into the pores of the synthetic graft, which will vary somewhat depending upon the pore sizes.

In place conjugate preparation continues by removing the synthetic graft from the crosslinking bath, preferably while retaining excess crosslinking solution thereon. Such graft is then exposed to a solution of anticoagulant material such as heparinous material, preferably one including highly active heparin fractions. Heparin exposure in this regard can be carried out within a bath including the heparinous material within a salt solution that is at a generally neutral or slightly alkaline pH. Excess heparinous material can be removed by rinsing with a solution on the order of the solvent or carrier liquid of the anticoagulant solution.

When it is desired to further include a cell attachment protein on the conjugate of the treated synthetic porous graft, the graft is treated with an appropriate solution of cell attachment protein such as one including purified human fibronectin. Preferably, this is accomplished by flowing the cell attachment protein solution through the lumen of a previously treated graft that is generally tubular.

The completed treated synthetic porous graft will, after rinsing, be stored under appropriate storage conditions as may be suitable for particular needs. Included are storage in a frozen state, in a dried state after lyophilization, as well as storage in different liquid solutions, for example 70% alcohol, 50% alcohol, glutaraldehyde, or mixtures thereof.

EXAMPLE I

Tubular synthetic porous grafts having internal diameters of 6 mm and 4 mm having pores of a size less than about 70 microns are made by winding extruded polyurethane fibers on a mandrel having respective outer diameters of 6 mm and 4 mm. These synthetic grafts are made on an apparatus generally in accordance with that shown in Figures 5, 6 and 7. One end of the length of graft material is tied off so as to substantially prevent solution flow through that end of the lumen. A low pH solution of type III collagen is syringe injected into the open end of the lumen of the length of synthetic graft until the solution is observed to ooze out through the porous graft substantially throughout its length. Excess collagen is removed by rinsing with the low pH carrier liquid, and the graft is immersed into a bath of concentrated salt solution, after which the type III collagen fibrils are reassociated in situ as the fibril forming solution diffuses throughout the graft.

Following a brief rinse with plain buffer solution, the graft is soaked in a slightly alkaline glutaraldehyde solution in order to effect crosslinking of the collagen fibers. Thereafter, the graft is very briefly rinsed with plain buffer and immersed into a heparin salt solution at a generally neutral pH, by virtue of which heparin is coupled to the collagen fibers. The thus formed treated synthetic porous graft is rinsed with salt solution and stored in an alcohol solution.

## EXAMPLE 2

Fibronectin solution is directed into the lumen of the treated synthetic grafts in accordance with Example I, and the lumen is clamped off to permit the fibronectin to attach to the conjugate around the luminal surface of the grafts. The fibronectin solution includes purified fibronectin to yield a relatively high concentration of deposited functional protein, approximately 5 micrograms per square centimeter. Excess fibronectin solution is removed by brief rinses with the carrier liquid, and the resulting grafts are stored in a 50% ethanol solution.

## EXAMPLE 3

Synthetic grafts are made generally in accordance with Example I and Example 2, except the injected low pH collagen solution contains a blend of type I collagen fibrils and of type III collagen fibrils.

## EXAMPLE 4

Synthetic grafts are made generally in accordance with Example I and Example 2, except the injected low pH collagen solution contains type I collagen fibrils.

It will be understood that the embodiments of the present invention which have been described are illustrative of some of the applications of the principles of the present invention. Numerous modifications may be made by those skilled in the art without departing from the true spirit and scope of the invention.

**Claims**

I. A biologically modified synthetic graft, comprising:

a body member made from synthetic material, said body member including a plurality of pores within said synthetic material;

a conjugate member within said pores for modifying the biological properties of the synthetic body member, said conjugate member including collagen material and heparinous material coupled by a crosslinking component, said collagen material including collagen fibrils that are coupled together by said crosslinking component into a collagen fibril network within said pores, said collagen fibril network of the conjugate member having been formed in place within said pores.

2. The biologically modified synthetic graft according to claim I, wherein said body member includes·a plurality of layers of biocompatible polymeric fiber material, said fiber layers having been formed by extrusion of a fiber-forming polymer through an orifice to form an elongated fiber that was wound on a mandrel to form a plurality of windings defining said plurality of layers, said plurality of windings defining a non-woven graft body having overlying fibers that intersect one another to form said pores and at least one porous wall that is conducive to tissue ingrowth thereinto.

3. The biologically modified synthetic graft according to claim I and/or 2, wherein said synthetic graft body is made from a polyurethane material.

4. The biologically modified synthetic graft according to any of claims I - 3, wherein said synthetic material body member is a polymeric material including said pores, said pores having been formed by eluting elutable particles from a mixture of the polymeric material and such elutable particles.

5. The biologically modified synthetic graft according to any of claims I - 4, wherein said pores have a size not greater than on the order of about 70 microns.

6. The biologically modified synthetic graft according to any of claims I - 5, wherein said collagen material of the conjugate member includes type III collagen.

7. The biologically modified synthetic graft according to any of claims I - 5, wherein said collagen material of the conjugate member includes type I collagen.

8. The biologically modifed synthetic graft according to any of claims I - 5, wherein said collagen material of the conjugate member includes type I collagen and type III collagen.

9. The biologically modified synthetic graft according to any of claims I - 8, wherein said heparinous material of the conjugate member is a highly active heparin fraction isolated by affinity chromatography on anti-thrombin III sepharose columns.

I0. The biologically modified synthetic graft according to any of claims I - 9, wherein said crosslinking component of the conjugate member is a multi-functional aldehyde.

II. The biologically modified synthetic graft according to any of claims I - I0, wherein said formed-in-place collagen network of the conjugate member had been formed from solubilized collagen materials placed within said pores and reassociated within said pores into said collagen fibrils.

12. The biologically modified synthetic graft according to any of claims 1 - 11, wherein said conjugate member further includes cell attachment protein, said cell attachment protein being generally on at least one surface of said body member.

13. The biologically modified synthetic graft according to claim 12, wherein said body member is generally tubular and includes a lumen to define a luminal surface of the conjugate member, and said cell attachment protein of the conjugate member is coupled to said luminal surface of the conjugate member.

14. The biologically modified synthetic graft according to claim 12 and/or 13, wherein said cell attachment protein of the conjugate member is fibronectin.

15. A method for manufacturing a biologically modified synthetic graft, comprising:

making a body member from synthetic material, said making procedure including forming a plurality of pores within said synthetic material;

dissolving collagen fibrils within a solvent in order to form a solution of collagen material;

passing said collagen solutions into said pores of the body member;

reassociating said collagen fibrils from said collagen solution within said pores by contacting said body member having collagen solution within its pores with a collagen fibril reassociating agent;

treating said resulting reassociated collagen fibrils within said pores with a crosslinking component whereby collagen fibrils are formed into a collagen fibril network within said pores; and

contacting anticoagulant material with said collagen fibrils and in the presence of said crosslinking agent to form a synthetic graft that is biologically modified with said conjugate.

16. The method according to claim 15, wherein said making procedure includes forming a generally tubular synthetic body member having a lumen, and wherein said collagen solution passing procedure includes tying off one end of said lumen and injecting said collagen solution into the other end of said lumen.

17. The method according to claim 15 and/or 16, wherein said collagen fibrils include type III collagen.

18. The method according to claim 15 and/or 16, wherein said collagen fibrils include type I collagen.

19. The method according to claim 15 and/or 16, wherein said collagen fibrils include type I and type III collagen.

20. The method according to any of claims 15 - 19, wherein said anticoagulant material includes a heparinous material.

21. The method according to claim 20, wherein said anticoagulant material includes a heparinous material that is a highly active heparin fraction isolated by affinity chromatography on anti-thrombin III sepharose columns.

22. The method according to any of claims 15 - 21, wherein said crosslinking agent is a dialdehyde.

23. The method according to any of claims 15 - 22, wherein said making procedure includes:

extruding a fiber-forming biocompatible polymeric material through an orifice in order to form an elongated fiber;

winding said elongated fiber on a mandrel such that the continuous fiber forms a plurality of windings that define a non-woven cylinder having overlying fibers that contact one another to form said pores and at least one porous surface that is conducive to tissue ingrowth thereinto.

24. The method according to any of claims 15 - 23, wherein said making procedure includes providing and shaping a mixture of polymeric material and elutable particles and eluting said elutable particles from the shaped polymer in order to form said pores.

25. The method according to any of claims 15 - 24, further including coupling a cell attachment protein to said conjugate.

26. A process for the improvement of in vivo compatibility between blood and an implanted synthetic porous graft, said process comprising forming a conjugate in place within the pores of the synthetic porous graft, said conjugate including collagen material and anticoagulant material coupled by a crosslinking component, said conjugate including collagen fibrils that are coupled together by said crosslinking component into a collagen fibril network.

27. The process according to claim 26, wherein said collagen material includes type III collagen.

28. The process according to claim 26 and/or 27, wherein said anticoagulant material is a highly active heparin fraction isolated by affinity chromatography on anti-thrombin III sepharose columns.

29. The process according to any of claims 26 - 28, wherein said procedure of forming the conjugate in place further includes coupling cell attachment protein to said conjugate network.

30. A conjugate for improving the biocompatibility of synthetic grafts, said conjugate having been made according to the process of claim 26, said anticoagulant material being a heparinous material.

31. The conjugate according to claim 30, wherein said collagen material includes type I collagen fibrils and type III collagen fibrils.

32. The conjugate according to claim 30 and/or 3l, wherein said heparinous material of the conjugate member is a highly active heparin fraction isolated by affinity chromatography on anti-thrombin III sepharose columns.

33. The conjugate according to any of claims 30 - 32, wherein said conjugate further includes cell attachment protein coupled to the conjugate through said crosslinking component.

FIG_1.

FIG_2.

FIG_3.

FIG_4.

FIG_5.

FIG_6.

FIG_7.